(19) Europäisches Patentamt
European Patent Office
Office européen des brevets

(11) Publication number: 0 195 433
B1

(12) EUROPEAN PATENT SPECIFICATION

(45) Date of publication of patent specification: 08.02.89

(51) Int. Cl.$^4$: C 07 C 149/24, C 07 C 149/243, C 07 C 148/00, A 61 K 31/16

(21) Application number: 86103713.3

(22) Date of filing: 19.03.86

# (54) Amide derivatives processes for preparing them and use thereof.

(30) Priority: 19.03.85 JP 55002/85

(43) Date of publication of application: 24.09.86 Bulletin 86/39

(45) Publication of the grant of the patent: 08.02.89 Bulletin 89/06

(84) Designated Contracting States: BE CH DE FR GB LI NL SE

(56) References cited:
EP-A-0 170 048
DE-B-1 445 716
GB-A-2 053 204

(73) Proprietor: GREEN CROSS CORPORATION
15-1, Imabashi 1-chome Higashi-ku Osaka-shi
Osaka (JP)

(72) Inventor: Yokoyama, Kazumasa
7-2-201, Terauchi 2-chome
Toyonaka-shi Osaka (JP)
Inventor: Iwai, Masakazu
5-18, Koyama 5-chome
Fujiidera-shi Osaka (JP)
Inventor: Naito, Youichiro
3-1 B46-904, Otokoyama Hachibou
Yahata-shi Kyoto (JP)
Inventor: Fukaya, Chikara
11-33-604, Kema-cho 2-chome Miyakojima-ku
Osaka-shi Osaka (JP)

(74) Representative: Hoffmann, Klaus, Dr. rer. nat. et al
Hoffmann . Eitle & Partner Patentanwälte
Arabellastrasse 4
D-8000 München 81 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 195 433 B1

Courier Press, Leamington Spa, England.

## Description

Background of the invention

The present invention relates to amide derivatives obtained from sulfur-containing amines and aliphatic acids, as well as a process for producing such derivatives.

Both of the alkylated heavy and light chains (H and L chains) in human serum IgG have been found to have strong activities against ulcers in digestive tracts and against inflammation. While various proposals have been made with a view to explaining the mechanism behind these activities, one established observation is that the marked activity of the H and L chains in inhibiting the secretion of gastric juice is involved in their action against ulcers in digestive tracts (T. Mimura, T. Torada et al; J. Pharm. Dyn. *6*, 397 (1983)). As for their antiinflammatory activity, it has been discovered that inhibition of permeation through blood vessels and that of leukocyte migration are involved in that phenomenon. Several researchers have suggested the involvement of the —SH groups at the hinge region of IgG as the active component of each of the H and L chains.

Summary of the invention

One object of the present invention is to provide a novel compound having activities against ulcers in digestive tracts and against inflammation.

Another object of the present invention is to provide a process for producing such novel compound.

In the past, the present inventors have conducted various researches with a view to producing compounds having activities against ulcers in digestive tracts and against inflammation. In the course of these efforts, the inventors successfully created amide derivatives by means of combining sulfur-containing amines with $C_5$—$C_{18}$ unsaturated aliphatic acids through amide bonds. Surprisingly enough, the amide derivatives created were found to have superior activities against ulcers in digestive tracts and against inflammation. The present invention has been accomplished of the basis of these findings.

Therefore, in one aspect, the present invention relates to an amide derivative having a sulfur-containing amine (i.e., an amino compound containing one or more sulfur atoms) combined with a $C_5$—$C_{18}$ unsaturated aliphatic acid by one or more amide bonds. Stated more specifically, the amide derivative is characterized in that the amino group in the sulfur-containing amino compound is bonded to the carboxyl group in $C_5$—$C_{18}$ unsaturated aliphatic acid by means of an amide bond.

If the amide derivative of the present invention contains an SH group, a compound wherein said SH group is alkylated is also included within the scope of the definition of the derivative (unless otherwise noted, the term "amide derivative" which will be used hereinafter shall include such SH-alkylated compounds). It should also be mentioned that the alkyl group in an alkylated compound may be a substituted alkyl group. Illustrative alkyl groups are listed below (the term "lower" used throughout this specification shall be construed as assuming the presence of 1 to 4 carbon atoms, unless otherwise noted).

(1) lower alkyl groups such as methyl, ethyl and n-propyl;
(2) N,N-di(lower alkyl)carbamido-lower alkyl groups such as N,N-diethyl-carbamidomethyl;
(3) lower alkoxycarbonyl-lower alkyl groups such as ethoxycarbonylmethyl and ethoxycarbonylethyl;
(4) carboxy-lower alkyl groups such as carboxymethyl and carboxyethyl;
(5) cyano-lower alkyl groups such as cyanomethyl;
(6) β-amino-lower alkyl groups such as $—CH_2CH_2NH_2$;
(7) benzoyl-lower alkyl groups such as $—CH_2COC_6H_5$;
(8) carboxamide-lower alkyl groups such as $—CH_2CONH_2$; and
(9) lower alkyl groups substituted with 5- or 6-membered nitrogen-containing heterocyclic rings which may optionally contain an oxygen atom such as piperidine, pyrrolidine, morpholine and imidazole being preferred substituents.

Any amino compounds having one or more sulfur atoms may be used as the sulfur-containing amine in the present invention without any particular limitation. Preferably one or two amino groups are present in the sulfur-containing amine. Specific examples of the sulfur-containing amine include cystine, cysteine, cystamine, cysteamine, taurine, methionine, ethionine and lanthionine, with cystamine and cysteamine being particularly preferred. The sulfur-containing amine may be a compound wherein the SH group present is substituted with one or more of the alkyl groups listed above.

Any unsaturated aliphatic acid that has 5—18 carbon atoms, preferably 8—13 carbon atoms, may be used in the present invention without any particular limitation. The unsaturated bond is preferably a double bond. There is no particular limitation on the number of unsaturated groups present in the unsaturated aliphatic acid but those having 1—3 unsaturated groups are preferable. There is also no particular limitation on the position of unsaturated groups.

Preferred unsaturated aliphatic acids are those which are represented by the following formula:

$$CH_3(CH_2)_mCH{=}CH(CH_2)_nCOOH$$

where m+n is an integer of 1—14, provided that m is preferably an integer of 1—9 and n is preferably 0 or 1.

Specific examples of the unsaturated aliphatic acid that may be used in the present invention include:

2-octenoic acid, $CH_3(CH_2)_4CH{=}CHCOOH$; 2-decenoic acid,

$CH_3(CH_2)_6CH=CHCOOH$; 2-undecenoic acid, $CH_3(CH_2)_7CH=CHCOOH$; and 2-tridecenoic acid, $CH_3(CH_2)_9CH=CHCOOH$.

In a preferred embodiment, the amide derivative of the present invention is a compound represented by the general formula (I):

$$CH_3(CH_2)_pCH=CH(CH_2)_qCONH—A—S—X \quad (I)$$

wherein p+q is an integer of 1—14; A is an alkylene group; X is a hydrogen atom, carboxamidoalkyl group or

$$CH_3(CH_2)_pCH=CH(CH_2)_qCONH—A—S—$$

(wherein p, q and A each has the same meaning as defined above).

In formula (I), p+q represents an integer of 1—14, preferably an integer between 1 and 9; p is preferably an integer of 1—14, with an integer between 4 and 9 being particularly preferable; q is preferably 0. The alkylene group denoted by A is preferably a straight- or branched-chain alkylene group having 1—3 carbon atoms, such as methylene or ethylene, with the ethylene being particularly preferred.

The amide derivative of the present invention may be produced by a variety of known amidation techniques; for example, the unsaturated aliphatic acid or a reactive derivative thereof may be reacted with the sulfur-containing amine, optionally in the presence of a dehydrating agent.

Illustrative reactive derivatives of the unsaturated aliphatic acid include acid halides (e.g. acid chloride and acid bromide), active esters, and acid anhydrides.

Examples of the dehydrating agent that may be used in the reaction include N,N'-disubstituted carbodiimides (e.g., N,N'-dicyclohexylcarbodiimide) and N,N'-carbonyldiimidazole.

The reaction between the saturated aliphatic acid or a reactive derivative thereof with the sulfur-containing amine may be carried out in the presence of an inert solvent. Suitable inert solvents include ethyl acetate, chloroform and ether. The reaction time generally ranges from 30 minutes to 4 hours, and the reaction temperature is typically within the range of about 0 to 50°C.

The unsaturated aliphatic acid is typically used in an amount ranging from about 1 to 3 moles per mole of the sulfur-containing amine. The dehydrating agent, if it is used at all, is preferably incorporated in the reaction system in an amount of about 1 to 3 moles per mole of the sulfur-containing amine.

A reactive derivative, say, an acid halide, of the unsaturated aliphatic acid is typically prepared by treating the aliphatic acid with phosphorus trichloride, phosphorus pentachloride or, more preferably, with thionyl chloride in a weight ratio of the acid to thionyl chloride which ranges from 1:1 to 1:5. The treatment is preferably accomplished by means of reaction at 40—80°C for a period of 4—29 hours. The resulting acid halide can be purified by a suitable technique such as distillation.

The amide derivative of the present invention may have an SH group; in this case, it may be converted to an alkylated form. If the amide derivative contains a disulfide (S—S) group, the latter may be reduced to an SH group before the derivative is converted to an alkylated form. Conversion of these particular types of the amide derivatives to the corresponding alkylated forms may be carried out by any known alkylation technique (e.g. Biochemistry, *7* (5), 1950—1958 (1968)).

The amide derivative synthesized by the procedures described above may be isolated and purified by any known technique such as phase transfer, recrystallization or chromatography.

The amide derivative of the present invention exhibits superior activities against ulcers in digestive tracts in mammals (e.g. humans, horses, dogs, mice and rats) and against inflammation in these animals. Therfore, this compound has potential utility for pharmaceuticals effective against ulcers or inflammations.

The amide derivative of the present invention may be administered either orally or parenterally in the form of a pharmaceutical preparation as combined with an appropriate and conventional pharmaceutically acceptable carrier. The preparation may assume any conventional dosage form such as a tablet, capsule, powder, suppository or an injection. For oral administration, the amide derivative of the present invention is typically administered in an amount of 10—100 mg/human daily in one or more doses. But it should be noted that the exact dose of the compound can vary with the age of the patient, his body weight and/or the severity of the disease to be treated or the patient's response to the therapy.

Experiment 1

(1) Activity in inhibiting the secretion of gastric juice

The activity of each of the test compounds in inhibiting the secretion of gastric juice was measured by pyloric ligation in accordance with the method of Shay et al. described in H. Shay, S. A. Komarov, S. S. Fels et al.; Gastroenterology, *5*, 43 (1945). Groups each consisting of 8—10 Wistar male rats weighing 150—200 g which had been starved for 48 hours were subjected to abdominal incision under ether anesthetization. After pyloric ligation, the incised abdomen of each animal was closed and the animal was kept away from food and water for 4 hours. The test compound was suspended in 5% gum arabic (in physiological saline) to give a concentration of 5 mg/ml, and the suspension was administered intraperitoneally to each rat in a dose of 25 mg/kg immediately after the closure of the incised abdomen. Four hours later, each rat was sacrificed with chloroform and the stomach was extracted. The gastric juice contained in the stomach was withdrawn and checked for its volume, total acidity and total pepsin activity.

The total acidity was determined by titration with 0.02N NaOH using phenolphthalein as an indicator. The total pepsin activity was determined in accordance with the method of Anson; Brit. J. Pharmacol., *13*, 54 (1958) using casein as a substrate. The results are shown in Table 1.

TABLE 1

| | Gastric volume | Total acid output | Total peptic activity |
|---|---|---|---|
| | (ml/100 g wt.) | (μ Eq/100 g wt.) | (mg as tyrosine/ 100 g wt.) |
| Control | 2.98 | 331.4 | 184.2 |
| 2-Undecenoic acid | 2.46 | 318.1 | 159.8 |
| Compound 1 | 2.10 | 229.2 | 139.9 |
| Compound 2 | 0.97 | 97.4 | 70.9 |
| Compound 6 | 0.93 | 91.1 | 81.5 |

(Dose of test compound: 25 mg/kg, i.p.)
Compound 1: $-[SCH_2CH_2NH-COCH=CH(CH_2)_7CH_3]_2$
Compound 2: $NH_2COCH_2SCH_2CH_2NH-COCH=CH(CH_2)_7CH_3$
Compound 6: $NH_2COCH_2SCH_2CH_2NH-COCH=CH(CH_2)_6CH_3$

(2) Aspirin ulcer

Aspirin ulcers were produced in Wistar male rats (body weight: 150—200 g) in accordance with the method described in S. Okabe, K. Takeuchi et al.; Jpn. J. Pharmacol., *24*, 363 (1974).

The rats were starved for 24 hours and subjected to pyloric ligation. Immediately thereafter, 150 mg/kg of aspirin suspended in 5% gum arabic was administered orally to each rat which was simultaneously injected with a selected test compound (20 mg/kg) intraperitoneally.

The rats were kept away from food and water for 7 hours. Thereafter, the stomach was extracted from each animal under ether anesthetization and fixed in formalin. The total length of the ulcers occurring in the glands of the extracted stomach was measured and used as an ulcer index.

(3) HCl-Ethanol ulcer

HCl-ethanol ulcers were induced in Wistar male rats in accordance with the method described in S. Okabe, S. Ueki et al.; Oyo Yakuri, *27*, 829 (1984).

The procedures of (2) were repeated except that 2 hours after the intraperitoneal injection of a selected test compound, 1.5 ml of 15 mM HCl in 60% ethanol was administered orally to each rat, which thereafter was kept for an additional 1 hour.

(4) Stress ulcer

Stress ulcers were induced in Wistar male rats in accordance with the method described in K. Takagi and S. Okabe; Jpn. J. Pharmacol., *18*, 9 (1968).

The procedures of (2) were repeated except that the rats were restrained immediately after the administration of a selected test compound and immersed in water (22±1°C) for 7 hours.

(5) Acetic acid ulcer

Acetic acid ulcers were induced in Wistar male rats in accordance with the method described in K. Takagi, S. Okabe et al.,; Jpn. J. Pharmacol., *19*, 418 (1969).

Starting on the day of operation, each of the test compounds was administered orally to each rat for 14 consecutive days on a two-dose-a-day basis. The rats were starved for 24 hours after administration of the final dose. They then were sacrificed with chloroform and the stomach was extracted from each animal and fixed in formalin. The product of the length and breadth of each stomach was determined and used as an ulcer index.

The results are summarized in Tables 2 and 3.

TABLE 2

| Ulcer type | Test compound | Ulcer index | Percent inhibition |
|---|---|---|---|
| Aspirin ulcer | Control | 17.1 | — |
| | Compound 2 | 4.0 | 76.4 |
| | Compound 6 | 8.6 | 49.7 |
| HCl-ethanol ulcer | Control | 41.3 | — |
| | Compound 2 | 12.2 | 70.5 |
| | Compound 6 | 12.5 | 69.7 |
| Stress ulcer | Control | 29.1 | — |
| | Compound 2 | 9.0 | 69.1 |
| | Compound 6 | 13.3 | 54.3 |

(Dose of test compound: 20 mg/kg, i.p.)
Compound 2: $NH_2COCH_2SCH_2CH_2NH—COCH=CH(CH_2)_7CH_3$
Compound 6: $NH_2COCH_2SCH_2CH_2NH—COCH=CH(CH_2)_6CH_3$

TABLE 3

| Test compound | Dose (mg/kg/day) | Ulcer index | Percent healing |
|---|---|---|---|
| Control | — | 58.0 | — |
| Compound 6 | 400×2 | 38.3 | 34.0 |
| Compound 6 | 200×2 | 42.1 | 27.4 |
| Compound 6 | 100×2 | 45.8 | 21.0 |

Compound 6: $NH_2COCH_2SCH_2CH_2NH—COCH=CH(CH_2)_6CH_3$

Experiment 2
(1) Anti-inflammatory activity as measured by the carrageenan paw edema method

SD male rats (body weight: 150—180 g) were starved overnight and 25 mg/kg of a selected test compound was administered intraperitoneally to each rat. Thirty minutes later, 0.1 ml of a 1.5% solution of carrageenan in physiological saline was injected under the skin of the paw of a hind leg. Four hours after injection, the volume of the treated leg was measured with a mercury manometer and compared with the value prior to carrageenan injection in order to calculate the percentage of edema and its inhibition. The results are summarized in Table 4. Each of the test compounds was administered in a concentration of 5 mg/ml as a suspension in 5% gum arabic in physiological saline.

TABLE 4

| Test compound | Percentage of edema | Percent inhibition |
|---|---|---|
| Control | 51.6 | — |
| Compound 3 | 36.3 | 29.7 |
| Compound 4 | 28.3 | 45.2 |
| Control | 48.2 | — |
| Compound 7 | 33.4 | 30.7 |
| Compound 8 | 24.3 | 49.6 |

(Dose of test compound: 25 mg/kg, i.p.)
Compound 3: $\text{+}SCH_2CH_2NH—COCH{=}CH(CH_2)_4CH_3]_2$
Compound 4: $NH_2COCH_2SCH_2CH_2NH—COCH{=}CH(CH_2)_4CH_3$
Compound 7: $\text{+}SCH_2CH_2NH—COCH{=}CH(CH_2)_9CH_3]_2$
Compound 8: $NH_2COCH_2SCH_2CH_2NH—COCH{=}CH(CH_2)_9CH_3$

(2) Anti-inflammatory Activity as measured by the granuloma pouch method

Pouches were made under the dorsal skin of SN male rats (body weight: 150—180 g) by injecting 20 ml of $N_2$ gas in accordance with the method described in E. Itoga and Y. Miyake; Oyo yakuri, *66*, 316 (1970). The rats were subsequently injected with 1 ml of 1% croton oil dissolved in cottonseed oil. Eight days after injection, the wet weight of each pouch and the volume of exudate therefrom were measured. Each of the test compounds was administered orally for 8 consecutive days on a single-dose-a-day basis. The results are shown in Table 5.

TABLE 5

| Test compound | Dose (mg/kg/day) | Weight of pouch (g) | Volume of exudate (ml) |
|---|---|---|---|
| Control | — | 5.70 | 12.0 |
| Compound 3 | 25 | 4.34 | 8.5 |
| Compound 4 | 25 | 3.81 | 10.6 |

Compound 3: $\text{+}SCH_2CH_2NH—COCH{=}CH(CH_2)_4CH_3]_2$
Compound 4: $NH_2COCH_2SCH_2CH_2NH—COCH{=}CH(CH_2)_4CH_3$

Example 1

Synthesis of Compound 1, $\text{+}SCH_2CH_2NHCOCH{=}CH(CH_2)_7CH_3]_2$

A mixture of 2-undecenoic acid (15 g) and thionyl chloride (20 ml) was heated under reflux for 15 hours. After the reaction, thionyl chloride was distilled off and the residue was distilled under vacuum to obtain an acid chloride (12.3 g, 74%, b.p. 102.5—107.5°C). A solution (15 ml) of this acid chloride (12.3 g) in ethyl acetate was added dropwise to the ice-cooled solution (150 ml) of cystamine (4.2 g) and triethylamine (8.5 ml) in ethyl acetate. Reaction was carried out for 30 minutes at 0°C, then overnight at room temperature. Water (100 ml) was added to the reaction solution and the solid was recovered by filtration. By recrystallization with ethyl acetate, compound 1 was obtained (9.14 g, 68%, m.p. 133—133.5°C).

Compound 1 had the following properties.

(1) Melting point: 133—133.5°C (in ethyl acetate)
(2) IR $\nu_{max}^{KBr}$: 3300, 3050, 2920, 2850, 1650, 1600, 1545, 975, 650 $cm^{-1}$
(3) NMR: $\delta(CDCl_3+CF_3COOH)$ 0.9 (brt, 6H), 2.4 (brq, 4H), 2.9 (t, 4H, J=7Hz), 3.8 (brt, 4H), 6.2 (d, 2H, J=16Hz), 7.2 (dt, J=16, 7Hz) 7.8 (br, 2H)

Example 2

Synthesis of compound 2, $H_2NCOCH_2SCH_2CH_2NH—COCH{=}CH(CH_2)_7CH_3$

Compound 1 (2.0 g) was suspended in 60 ml of a 9:1 mixture of methanol and water, and tributyl phosphine (1.13 ml) was added dropwise to the suspension in a nitrogen atmosphere. Reaction was carried

out for 30 minutes at 0°C, and for an additional 1 hour at room temperature. Iodoacetamide (1.6 g) was added to the reaction mixture and 1N NaOH (8.7 ml) was added dropwise to the cooled mixture. Thereafter, the mixture was agitated for 1 hour at room temperature. Water (100 ml) was added to the reaction solution and the white precipitate was recovered by filtration. By recrystallization from ethanol-water, Compound 2 was obtained (2.0 g, 81%, m.p. 150.5—151.0°C).

Compound 2 had the following properties.

(1) Melting point: 150.5—151.0°C (in ethanol-water)

(2) IR $\nu_{max}^{KBr}$: 3350, 3300, 3150, 2950, 2850, 1650, 1620, 1540, 1460, 970 $cm^{-1}$

(3) NMR: δ(DMSO-$d_6$+$CDCl_3$ 0.9 (br, t, 3H), 2.1 (br, 2H), 2.7 (t, 2H, J=7Hz), 3.3 (s, 2H), 3.35 (q, 2H, J=7Hz), 5.85 (d, 1H, J=16Hz), 6.7 (dt, J=16, 7Hz), 7.2 (br, 1H), 7.9 (br, 2H)

Example 3

Synthesis of Compound 3, $-[SCH_2CH_2NH—COCH=CH(CH_2)_4CH_3]_2$

Using 2-octenoic acid (3.9 g, 27.6 mmol), the procedures of Example 1 were repeated to obtain Compound 3 (3.4 g, 85%).

This compound had the following properties.

(1) Melting point: 131—133°C

(2) IR $\nu_{max}^{KBr}$: 3300, 3050, 2920, 2850, 1650, 1620, 1445, 970 $cm^{-1}$

(3) NMR: δ($CDCl_3$+$CF_3COOH$) 0.9 (brt, 6H), 2.15 (brt, 4H) 2.85 (t, 4H, J=7Hz) 3.6 (q, 4H, J=7Hz) 5.9 (dt, 2H, J=16, 1Hz) 5.8 (dt, 2H, J=16, 7Hz), 5.9 (br, 2H)

Example 4

Synthesis of Compound 4, $NH_2COCH_2SCH_2CH_2NH—COCH=CH(CH_2)_4CH_3$

Using Compound 3 (1.0 g, 2.5 mmol) obtained in Example 3, the procedures of Example 2 were repeated to obtain Compound 4 (1.0 g, 77%).

This compound had the following properties.

(1) Melting point: 146.5—147°C

(2) IR $\nu_{max}^{KBr}$: 3350, 3300, 3150, 2920, 2850, 1635, 1600, 1540, 970 $cm^{-1}$

(3) NMR: δ($CDCl_3$+DMSO-$d_6$) 0.9 (brt, 3H), 2.1 (br, 2H), 2.65 (t, 2H, J=7Hz), 3.3 (s, 2H), 3.35 (q, 2H, J=7Hz) 5.8 (d, 1H, J=16Hz), 6.7 (dt, 1H, J=16, 7Hz), 7.4 (br, 1H), 7.9 (br, 2H)

Example 5

Synthesis of Compound 5, $-[SCH_2CH_2NH—COCH=CH(CH_2)_6CH_3]_2$

Using 2-decenoic acid (15 g), the procedures of Example 1 were repeated to obtain Compound 5 (10.75 g, 75%).

This compound had the following properties.

(1) Melting point: 136—136.5°C

(2) IR $\nu_{max}^{KBr}$: 3300, 3050, 2920, 2850, 1665, 1620, 1540, 970 $cm^{-1}$

(3) NMR: δ($CDCl_3$+$CF_3COOH$) 0.9 (br, 6H), 2.4 (br, 4H) 2.9 (t, 4H, J=7Hz), 3.7 (t, 4H, J=7Hz) 6.1 (d, 2H, J=15Hz), 7.1 (dt, 2H, J=15, 6Hz) 7.2 (br, 2H)

Example 6

Synthesis of Compound 6, $NH_2COCH_2SCH_2CH_2NH—COCH=CH(CH_2)_6CH_3$

Using Compound 5 (2.0 g, 4.38 mmol), the procedures of Example 2 were repeated to obtain Compound 6 (2.1 g, 84%).

This compound had the following properties.

(1) Melting point: 151—151.5°C

(2) IR $\nu_{max}^{KBr}$: 3350, 3300, 3150, 2920, 2850, 1650, 1620, 1540, 970 $cm^{-1}$

(3) NMR: δ(DMSO-$d_6$+$CDCl_3$) 0.9 (brt, 3H), 2.1 (brt, 2H), 2.65 (t, 2H, J=6Hz), 3.1 (s, 2H), 3.4 (q, 2H, J=6Hz), 5.8 (d, 1H, J=16Hz), 6.7 (dt, 1H, J=16, 7Hz), 6.9 (br, 1H), 7.3 (br, 1H), 7.9 (br, 1H)

Example 7

Synthesis of Compound 7, $-[SCH_2CH_2NH—COCH=CH(CH_2)_9CH_3]_2$

Using 2-tridecenoic acid (8.4 g), the procedures of Example 1 were repeated to obtain compound 7 (3.28 g, 67%).

This compound had the following properties.

(1) Melting point: 131—134°C

(2) IR $\nu_{max}^{KBr}$: 3300, 3050, 2950, 2850, 1670, 1630, 1550, 980 $cm^{-1}$

(3) NMR: δ($CDCl_3$+$CF_3COOH$) 0.9 (br, 6H), 2.3 (br, 4H), 2.9 (br, 4H) 3.9 (br, 4H), 6.1 (brd, 2H, J=15Hz) 7.2 (br, 2H)

Example 8

Synthesis of Compound 8, $H_2NCOCH_2SCH_2CH_2NHCOCH=CH(CH_2)_9CH_3$

Using Compound 7 (3.7 g, 6.85 mmol), the procedures of Example 2 were repeated to obtain Compound 8 (2.04 g).

This compound had the following properties.
(1) Melting point: 151—152°C
(2) IR $\nu_{max}^{KBr}$: 3400, 3350, 3200, 2950, 2850, 1650, 1630, 1450, 1460, 980 $cm^{-1}$
(3) NMR: δ($CDCl_3$+$CF_3COOH$) 0.9 (br, 3H), 2.4 (br, 2H), 3.0 (br, 2H) 3.5 (s, 2H), 3.8 (br, 2H), 6.1 (brd, 1H, J=15Hz) 7.0—7.9 (br, m, 4H)

**Claims**

1. An amide derivative represented by the general formula (I):

$$CH_3(CH_2)_pCH=CH(CH_2)_qCONH—A—S—X \quad (I)$$

wherein p+q is an integer of 1—14; A is an alkylene group or a carboxy substituted alkylene group; X is a hydrogen atom, a substituted or unsubstituted alkyl group, a carboxamide-alkyl group or

$$CH_3(CH_2)_pCH=CH(CH_2)_qCONH—A—S—$$

wherein p, q and A each has the same meaning as defined above.

2. An amide derivative according to claim 1, wherein X is hydrogen.

3. An amide derivative according to claim 1, wherein X is an alkyl group having 1 to 4 carbon atoms which is unsubstituted or substituted.

4. An amide derivative according to claim 3, wherein said alkyl group is substituted with an N,N-di(lower alkyl) carbamidoalkyl group, a lower alkoxycarbonyl-lower alkyl group, a carboxy-lower alkyl group, a cyano-lower alkyl group, a β-amino-lower alkyl group, a benzoyl-lower alkyl group, a carboxamide-lower alkyl group or a nitrogen-containing heterocyclic-lower alkyl group.

5. An amide derivative according to claim 1, wherein the moiety —NH—A—S—X represents an N substituted sulfur-containing amine selected from cystine, cystein, cystamine, cysteamine, taurine, methionine, ethionine or lanthionine, or an alkylated derivative thereof.

6. An amide derivative according to claim 5, wherein the moiety —NH—A—S—X represents an N substituted sulfur-containing amine selected from cystamine or an alkylated cysteamine.

7. An amide derivative according to claim 5 or claim 6, wherein said alkylated derivative contains an N,N-di(lower alkyl)carbamidoalkyl group, a lower alkoxycarbonyl-lower alkyl group, a carboxy-lower alkyl group, a cyano-lower alkyl group, a β-amino-lower alkyl group, a benzoyl-lower alkyl group, a carboxamide-lower alkyl group or a nitrogen-containing heterocyclic-lower alkyl group.

8. A pharmaceutical composition comprising an amide derivative according to any preceding claim and a pharmaceutically acceptable carrier.

9. An amide derivative according to any of claims 1 to 7 for use as a medicament.

10. An amide derivative according to any of claims 1 to 7 for use as an anti-ulcer or anti-inflammatory agent.

11. A process for preparing a compound of formula (I):

$$CH_3(CH_2)_pCH=CH(CH_2)_qCONH—A—S—X \quad (I)$$

wherein p+q is an integer of 1—14; A is an alkylene group or a carboxy substituted alkylene group; and X is a hydrogen atom, a substituted or unsubstituted alkyl group, a carboxamide-alkyl group or

$$CH_3(CH_2)_pCH=CH(CH_2)_qCONH—A—S—$$

wherein p, q and A each has the same meaning as defined above, comprising the step of reacting a compound of formula (II):

$$CH_3(CH_2)_pCH=CH(CH_2)_qCO_2H \quad (II)$$

wherein p+q is as defined above, or a reactive derivative thereof with a compound of formula (III):

$$H_2N—A—S—Y \quad (III)$$

wherein Y is X as defined above or a group —S—A—$NH_2$, and A is as defined above.

12. A process according to claim 11, wherein the compound of formula III is selected from cystine, cystein, cystamine, cysteamine, taurine, methionine, ethionine or lanthionine or an alkylated derivative thereof.

13. A process according to claim 11 or claim 12, wherein said reactive derivative of compound II is an acid chloride, acid bromide, acid anhydride or reactive ester.

14. A process according to any of claims 11 to 13, wherein the reaction is carried out in the presence of a dehydrating agent.

15. A process according to claim 14, wherein said dehydrating agent is N,N'-dicyclohexyl-carbodiimide or N,N'-carboxyldiimidazole.

16. A process for preparing a compound of formula (Ia):

$$CH_3(CH_2)_pCH{=}CH(CH_2)_qCONH{-}A{-}S{-}H \quad (Ia)$$

wherein p+q is an integer of 1—14; and A is an alkylene group or a carboxy substituted alkylene group, comprising the step of reducing a compound of formula (IV):

$$[CH_3(CH_2)_pCH{=}CH(CH_2)_qCONH{-}A{-}S{-}]_2$$

wherein A and p+q are as defined above.

17. A process for preparing a compound of formula (Ib):

$$CH_3(CH_2)_pCH{=}CH(CH_2)_qCONH{-}A{-}S{-}X \quad (Ib)$$

wherein p+q is an integer of 1—14; A is an alkylene group or a carboxy substituted alkylene group; and X is a substituted or unsubstituted alkyl group having 1 to 4 carbon atoms, comprising the step of alkylating a compound of formula (Ia):

$$CH_3(CH_2)_pCH{=}CH(CH_2)_qCONH{-}A{-}S{-}H$$

wherein A and p+q are as defined above.

18. Une of an amide derivative according to any of claims 1 to 7 for the preparation of a medicament for the treatment of ulcers in digestive tracts and inflammation.

**Patentansprüche**

1. Amidderivat der allgemeinen Formel (I)

$$CH_3(CH_2)_pCH{=}CH(CH_2)_qCONH{-}A{-}S{-}X \quad (I)$$

wobei p+q eine Zahl von 1 bis 14 darstellt, A ist eine Alkylengruppe oder eine Carboxy-substituierte Alkylengruppe; X ist ein Wasserstoffatom, eine substituierte oder unsubstituierte Alkylgruppe, eine Carboxamid-alkylgruppe oder

$$CH_3(CH_2)_pCH{=}CH(CH_2)_qCONH{-}A{-}S{-},$$

wobei p, q und A jeweils die gleiche Bedeutung wie oben definiert besitzen.

2. Amidderivat nach Anspruch 1, wobei X Wasserstoff ist.

3. Amidderivat nach Anspruch 1, wobei X eine Alkylgruppe mit 1 bis 4 Kohlenstoffatomen, die unsubstituiert oder substituiert ist, darstellt.

4. Amidderivat nach Anspruch 3, wobei die Alkylgruppe mit einer N,N - Di(niedrigalkyl)carbamidoalkylgruppe, einer Niedrigalkoxycarbonyl - niedrigalkylgruppe, einer Carboxy - niedrigalkylgruppe, einer Cyano - niedrigalkylgruppe, einer β - Amino - niedrigalkylgruppe, einer Benzoyl - niedrigalkylgruppe, einer Carboxamid - niedrigalkylgruppe oder einer stickstoffhaltigen, heterozyklischen Niedrigalkylgruppe substituiert ist.

5. Amidderivat nach Anspruch 1, wobei der Teil —NH—A—S—X ein N-substituiertes, schwefelhaltiges Amin, ausgewählt aus Cystin, Cystein, Cystamin, Cysteamin, Taurin, Methionin, Ethionin oder Lanthionin, oder ein alkyliertes Derivat davon darstellt.

6. Amidderivat nach Anspruch 5, wobei der Teil —NH—A—S—X ein N-substituiertes, schwefelhaltiges Amin, ausgewählt aus Cystamin oder einem alkylierten Cysteamin, darstellt.

7. Amidderivat nach Anspruch 5 oder 6, wobei das alkylierte Derivat eine N,N - Di(niedrigalkyl)carbamidoalkylgruppe, eine Niedrigalkoxycarbonyl - niedrigalkylgruppe, eine Carboxy - niedrigalkylgruppe, eine Cyano - niedrigalkylgruppe, eine β - Amino - niedrigalkylgruppe, eine Benzoyl - niedrigalkylgruppe, eine Carboxamid - niedrigalkylgruppe oder eine stickstoffhaltige, heterozyklische Niedrigalkylgruppe enthält.

8. Pharmazeutische Zusammensetzung mit einem Amidderivat nach einem der vorhergehenden Ansprüche und einem pharmazeutisch verträglichen Träger.

9. Amidderivat nach einem der Ansprüche 1 bis 7 zur Verwendung als ein Arzneitmittel.

10. Amidderivat nach einem der Ansprüche 1 bis 7 zur Verwendung als ein Antigeschwür- oder entzündungshemmendes Mittel.

11. Verfahren zur Herstellung einer Verbindung der Formel (I)

$$CH_3(CH_2)_pCH{=}CH(CH_2)_qCONH{-}A{-}S{-}X \quad (I)$$

wobei p+q eine Zahl von 1 bis 14 dedeutet; A ist eine Alkylengruppe oder eine Carboxy-substituierte Alkylengruppe; und X ist ein Wasserstoffatom, eine substituierte oder unsubstituie-te Alkylgruppe, eine Carboxamid-alkylgruppe oder

$$CH_3(CH_2)_pCH{=}CH(CH_2)_qCONH{-}A{-}S{-},$$

wobei p, q und A jeweils die gleiche Bedeutung wie oben definiert besitzen, das die Reaktion einer Verbindung der Formel (II)

$$CH_3(CH_2)_pCH{=}CH(CH_2)_qCO_2H \qquad (II)$$

wobei p+q wie oben definiert ist, oder eines reaktiven Derivates davon mit einer Verbindung der Formel (III)

$$H_2N{-}A{-}S{-}Y \qquad (III)$$

wobei Y das oben definierte X oder eine Gruppe $-S-A-NH_2$ darstellt, und A wie oben definiert ist, umfasst.

12. Verfahren nach Anspruch 11, wobei die Verbindung der Formel (III) aus Cystin, Cystein, Cystamin, Cysteamin, Taurin, Methionin, Ethionin oder Lanthionin oder einem alkylierten Derivat davon ausgewählt wird.

13. Verfahren nach Anspruch 11 oder 12, wobei das reaktive Derivat der Verbindung (II) ein Säurechlorid, Säurebromid, Säureanhydrid oder ein reaktiver Ester ist.

14. Verfahren nach einem der Ansprüche 11 bis 13, wobei die Reaktion in Gegenwart eines Dehydratisierungsmittels ausgeführt wird.

15. Verfahren nach Anspruch 14, wobei das Dehydratisierungsmittel N,N' - Dicyclohexyl - carbodiimid oder N,N' - Carboxyldiimidazol ist.

16. Verfahren zur Herstellung einer Verbindung der Formel (Ia)

$$CH_3(CH_2)_pCH{=}CH(CH_2)_qCONH{-}A{-}S{-}H \qquad (Ia)$$

wobei p+q eine Zahl von 1 bis 14 darstellt; und A eine Alkylengruppe oder eine Carboxy-substituierte Alkylengruppe ist, das die Reduktion einer Verbindung der Formel (IV)

$$[CH_3(CH_2)_pCH{=}CH(CH_2)_qCONH{-}A{-}S{-}]_2$$

wobei A und p+q wie oben definiert sind, umfasst.

17. Verfahren zur Herstellung einer Verbindung der Formel (Ib)

$$CH_3(CH_2)_pCH{=}CH(CH_2)_qCONH{-}A{-}S{-}X \qquad (Ib)$$

wobei p+q eine Zahl von 1 bis 14 darstellt; A ist eine Alkylengruppe oder eine Carboxy-substituierte Alkylengruppe; und X ist eine substituierte oder unsubstituierte Alkylgruppe mit 1 bis 4 Kohlenstoffatomen, das die Alkylierung einer Verbindung der formel (Ia)

$$CH_3(CH_2)_pCH{=}CH(CH_2)_qCONH{-}A{-}S{-}H \qquad (Ia)$$

wobei A und p+q wie oben definiert sind, umfasst.

18. Verwendung eines Amidderivates gemäss einem der Ansprüche 1 bis 7 zur Herstellung eines Arzneimittels zur Behandlung von Geschwütsten im Verdauungstrakt und zur Behandlung von Entzündungen.

**Revendications**

1. Un dérivé d'amide représenté par la formule générale (I)

$$CH_3(CH_2)_pCH{=}CH(CH_2)_qCONH{-}A{-}S{-}X \qquad (I)$$

dans laquelle p+q est un nombre entier de 1—14; A est un groupe alkylène ou un groupe alkylène substitué par un carboxy; X est un atome d'hydrogène, un groupe alkyle substitué ou non substitué, un groupe carboxamide-alkyle ou

$$CH_3(CH_2)_pCH{=}CH(CH_2)_qCONH{-}A{-}S{-}$$

où p, q et A ont chacun la même signification que précédemment.

2. Un dérivé d'amide selon la revendication 1, dans lequel X est de l'hydrogène.

3. Un dérivé d'amide selon la revendication 1, dans lequel X est un groupe alkyle ayant 1 à 4 atomes de carbone qui est substitué ou non substitué.

4. Un dérivé d'amide selon la revendication 3, selon lequel ledit groupe alkyle est substitué par un groupe N,N - di(alkyl inférieur)carbamino-alkyle, un groupe alcoxy inférieur carbonylalkyle inférieur, un groupe carboxy-alkyle inférieur, un groupe cyano-alkyle inférieur, un groupe β-amino-alkyle inférieur, un groupe benzoyl-alkyle inférieur, un groupe carboxamide-alkyle inférieur ou un groupe hétérocyclique contenant de l'azote-alkyle inférieur.

5. Un dérivé d'amide selon la revendication 1, selon lequel la portion —NH—A—S—X représente une amine contenant du soufre N-substituée choisis parmi les suivantes: cystine, cystéine, cystamine, cystéamine, teurine, méthionine, éthionine ou lanthionine, ou un dérivé alkylé de celles-ci.

6. Un dérivé d'amide selon la revendication 5, selon lequel la portion —NH—A—S—X représente une amine contenant du soufre N-substituée choisie parmi la cystamine ou une cystéamine alkylée.

7. Un dérivé d'amide selon la revendication 5 ou 6, selon lequel ledit dérivé alkylé contient un groupe N,N-di(alkyl intérieur)carbamido-alkyle, un groupe alcoxy inférieur carbonylalkyle inférieur, un groupe carboxy-alkyle inférieur, un groupe cyano-alkyle inférieur, un groupe β-amino-alkyle inférieur, un groupe benzoyl-alkyle inférieur, un groupe carboxyamide-alkyle inférieur ou un groupe hétérocyclique contenant de l'azote-alkyle inférieur.

8. Une composition pharmaceutique comprenant un dérivé d'amide selon l'une quelconque des revendications précédentes et un support pharmaceutique acceptable.

9. Un dérivé d'amide selon l'une quelconque des revendications 1 à 7 à utiliser comme médicament.

10. Un dérivé d'amine selon l'une quelconque des revendications 1 à 7 à utiliser comme agent anti-ulcère ou agent antiinflammatoire.

11. Un procédé de préparation d'un composé de formule (I):

$$CH_3(CH_2)_pCH{=}CH(CH_2)_qCONH{-}A{-}S{-}X{-} \qquad (I)$$

dans laquelle p+q est un nombre entier de 1—14; A est un groupe alkylène ou un groupe alkylène substitué par un carboxy; et X est un atome d'hydrogène, un groupe alkyle substitué ou non substitué, un groupe carboxamide-alkyle ou

$$CH_3(CH_2)_pCH{=}CH(CH_2)_qCONH{-}A{-}S,$$

où p, q et A ont chacun la même signification que mentionnée ci-dessus, comprenant l'étape de réaction d'un composé de formule (II):

$$CH_3(CH_2)_pCH{=}CH(CH_2)_qCO_2H \qquad (II)$$

dans laquelle p+q est comme défini précédemment, ou un dérivé réactif de ce dernier avec un composé de formule (III):

$$H_2N{-}A{-}S{-}Y \qquad (III)$$

dans laquelle Y est X comme défini précédemment ou un groupe —S—A—$NH_2$, et A est comme défini précédemment.

12. Un procédé selon la revendication 11, selon lequel ls composé de formule (III) est choisi parmi les suivants: cystine, cystéine, cystamine, cystéamine, taurine, méthionine, éthionine ou lanthionine ou un dérivé alkylé de celles-ci.

13. Un procédé selon la revendication 11 ou 12, selon lequel ledit dérivé réactif de composé (II) est un chlorure d'acide, un bromure d'acide, un anhydride d'acide ou un ester réactif.

14. Un procédé selon l'une quelconque des revendications 11 à 13, selon lequel la réaction est conduite en présence d'un agent déshydratant.

15. Un procédé selon la revendication 14, selon lequel ledit agent déshydratant est le N,N' - dicyclohexyl - carbodiimide ou le N,N' - carboxyldiimidazole.

16. Un procédé de préparation d'un composé de formule (Ia):

$$CH_3(CH_2)_pCH{=}CH(CH_2)_qCONH{-}A{-}S{-}H{-} \qquad (Ia)$$

dans laquelle p+q est un nombre entier de 1—14; et A est un groupe alkylène ou un groupe alkylène substitué par un carboxy, comprenant l'étape de réduction d'un composé de formule (IV):

$$[CH_3(CH_2)_pCH{=}CH(CH_2)_qCONH{-}A{-}S{-}]_2$$

dans laquelle A et p+q sont comme défini précédemment.

17. Un procédé de préparation d'un composé de formule (Ib):

$$CH_3(CH_2)_pCH=CH(CH_2)_qCONH—A—S—X— \quad (Ib)$$

dans laquelle p+q est un nombre entier de 1—14; A est un groupe alkylène ou un groupe alkylène substitué par un carboxy; et X est un groupe alkyle substitué ou non substitué ayant de 1 à 4 atomes de carbone, comprenant l'étape d'alkylation d'un composé de formule (Ia):

$$CH_3(CH_2)_pCH=CH(CH_2)_qCONH—A—S—H$$

dans laquelle A et p+q sont comme défini précédemment.

18. Utilisation d'un dérivé d'amide selon l'une quelconque des revendications 1 à 7 pour la préparation d'un médicament pour le traitement des ulcères dans les voies digestives et de l'inflammation.